# EUROPEAN PATENT APPLICATION

(11) **EP 3 679 854 A1**
(43) Date of publication of application: **15.07.2020**
(21) Application number: 18850169.6
(22) Date of filing: 03.09.2018
(51) Int. Cl.: A61B 3/10, A61B 3/15

(54) **SCANNING TYPE OCULAR FUNDUS IMAGING DEVICE**

(30) Priority: 04.09.2017 JP 2017169819
(71) Applicant: Kowa Company Ltd., Naka-ku Nagoya-shi Aichi 460-8625 (JP)
(72) Inventor: SUZUKI, Takayoshi, Chofu-shi, Tokyo 182-0021 (JP)
(74) Representative: LKGLOBAL Lorenz & Kopf PartG mbB Patentanwälte
(86) International application number: PCT/JP2018/032562
(87) International publication number: WO 2019/045094

(57) **Abstract**

Light from a laser light source (10) is projected on the ocular fundus of a subject eye via a scanning optical system (2) and an objective lens optical system (3), and reflected light from the ocular fundus follows the opposite path and is incident on a light shielding member (41) at which harmful reflected light from an objective lens plane is shielded. The reflected light from the ocular fundus having passed through the light shielding member is received by a light receiving element (43) via a light receiving pinhole (42) disposed in the vicinity of the ocular fundus conjugate position. The objective lens optical system is composed of a first lens group (30) and a second lens group (31), and within a normal diopter adjustment range, an ocular fundus conjugate plane (32) is moved in a space between the first and second lens groups so as not overlap the objective lens plane.

## Description

### [Technical Field]

The present invention relates to a scanning type ocular fundus imaging device that two-dimensionally scans and projects laser light on an ocular fundus and receives reflected light from the ocular fundus to capture an image of the ocular fundus.

### [Background Art]

A scanning type ocular fundus imaging device is known which two-dimensionally scans laser light in a horizontal direction and a vertical direction, illuminates an ocular fundus with the two-dimensionally scanned laser light, and receives reflected light from the ocular fundus to capture an image of the ocular fundus.

In such a scanning type ocular fundus imaging device, the light ray illuminating the ocular fundus is reflected from the surface of an objective lens and incident on a light receiving element, so that a center spot image due to the reflected light occurs at the center of the captured image of the subject eye and deteriorates the image quality. In a proposed configuration to eliminate such deterioration, a light shielding member is disposed at a position at which the image of a laser irradiation area on the objective lens plane is formed, harmful reflected light from the lens plane of the objective lens is prevented from being incident on the light receiving element, and an image of the ocular fundus is captured with high image quality (the following Patent Document 1).

### [Prior Art Documents]

### [Patent Documents]

[Patent Document 1] JP2016-28687A

### [Summary of the Invention]

### [Problems to be solved by the Invention]

In general, the objective lens is composed of a plurality of lenses rather than a single lens, and particularly in a wide-angle scanning type ocular fundus imaging device, the objective lens is composed of a lens group having three or more lenses. In such a scanning type ocular fundus imaging device, the focal length of an objective lens is shorter than the thickness, the ocular fundus conjugate plane may overlap any of lens planes of the lens group, and the reflected light generated at the lens plane overlapping the ocular fundus conjugate plane becomes harmful light (center spot image) to deteriorate the captured image quality.

Moreover, when performing diopter adjustment, the ocular fundus conjugate plane overlaps any of lens planes of the objective lens group depending on the diopter of the subject eye, thus causing the same problem.

The present invention has been made to solve such a problem, and an object of the present invention is to provide a scanning type ocular fundus imaging device that can capture an image of an ocular fundus with high image quality by shielding the harmful reflected light ray due to the reflection from a lens plane of an objective lens composed of a plurality of lenses.

### [Means for solving the Problems]

The present invention (claim 1) provides a scanning type ocular fundus imaging device comprising:
a light projecting optical system that projects light from a light source on an ocular fundus of a subject eye via an objective lens optical system, the light being scanned as scanned illumination light using a scanning optical system;
a light receiving optical system that receives reflected light from the ocular fundus via the objective lens optical system and the scanning optical system;
a diopter adjusting mechanism that moves an ocular fundus conjugate plane formed by the objective lens optical system in an optical axis direction;
a light receiving pinhole disposed in the vicinity of an ocular fundus conjugate position in the light receiving optical system; and
a light shielding member disposed in the light receiving optical system to shield harmful reflected light from a lens plane of the objective lens optical system,
wherein the objective lens optical system is composed of a first lens group and a second lens group disposed at a distance from the first lens group in the optical axis direction, and the ocular fundus conjugate plane is located between the first and second lens groups of the objective lens optical system within a diopter adjustment range by the diopter adjusting mechanism.

The present invention (claim 10, 11) provides a scanning type ocular fundus imaging device comprising:
a light projecting optical system that projects light from a light source on an ocular fundus of a subject eye via an objective lens optical system, the light being scanned as scanned illumination light using a scanning optical system;
a light receiving optical system that receives reflected light from the ocular fundus via the objective lens optical system and the scanning optical system;
a light receiving pinhole disposed in the vicinity of an ocular fundus conjugate position in the light receiving optical system; and
a light shielding member disposed in the light receiving optical system to shield harmful reflected light from a lens plane of the objective lens optical system.

In the invention of claim 10, the light receiving optical system and the objective lens optical system are configured such that a light beam of scanned back-projection light is separated on the lens plane of the objective lens optical system from a light beam of the scanned illumination light, wherein the scanned back-projection light is scanned by the scanning optical system after exiting the light receiving pinhole and passing through the light shielding member, wherein the same scanning as that for the scanned back-projection light is performed for the scanned illumination light, while in the invention of claim 11, the light receiving optical system and the objective lens optical system are configured such that a light beam of scanned back-projection light is separated on the ocular fundus conjugate plane from a light beam of reflected light of the scanned illumination light from the lens plane of the objective lens optical system, wherein the scanned back-projection light is scanned by the scanning optical system after exiting the light receiving pinhole and passing through the light shielding member, wherein the same scanning as that for the scanned back-projection light is performed for the scanned illumination light.

### [Advantageous Effect of the Invention]

In the present invention (claim 1), the ocular fundus conjugate plane is located in a space between the first lens group and second lens group of the objective lens optical system; therefore, the ocular fundus conjugate plane does not overlap a lens plane of the first or second lens group and it is possible to effectively prevent the occurrence of a center spot image due to harmful reflected light from the objective lens plane.

Moreover, in the present invention (claim 10), on a lens plane of the objective lens optical system, the light beam of the scanned back-projection light scanned by the scanning optical system after exiting the light receiving pinhole and passing through the light shielding member does not overlap the light beam of the scanned illumination light for which the same scanning as that for the scanned back-projection light is performed, and it is therefore possible to reliably prevent the occurrence of a center spot image.

Furthermore, in the present invention (claim 11), on the ocular fundus conjugate plane, the light beam of the scanned back-projection light scanned by the scanning optical system after exiting the light receiving pinhole and passing through the light shielding member does not overlap the light beam of the reflected light of the scanned illumination light from a lens plane of the objective lens optical system, for which the same scanning as that for the scanned back-projection light is performed, and it is therefore possible to reliably prevent the occurrence of a center spot image.

### [Brief Description of Drawings]

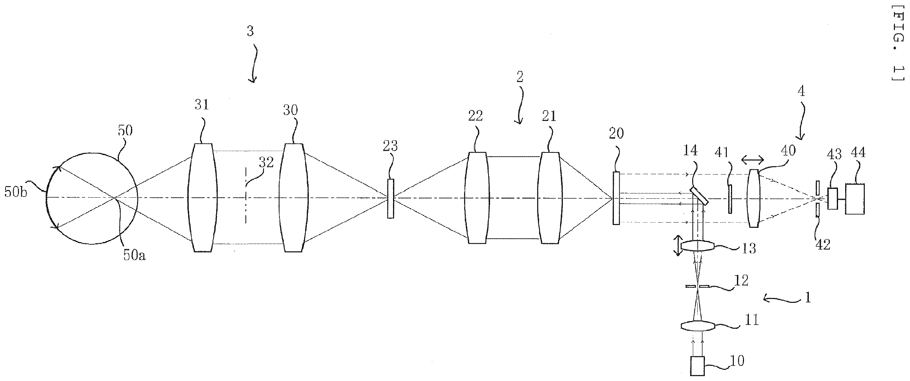

FIG. 1 is an optical diagram illustrating the optical configuration of a scanning type ocular fundus imaging device as a whole.

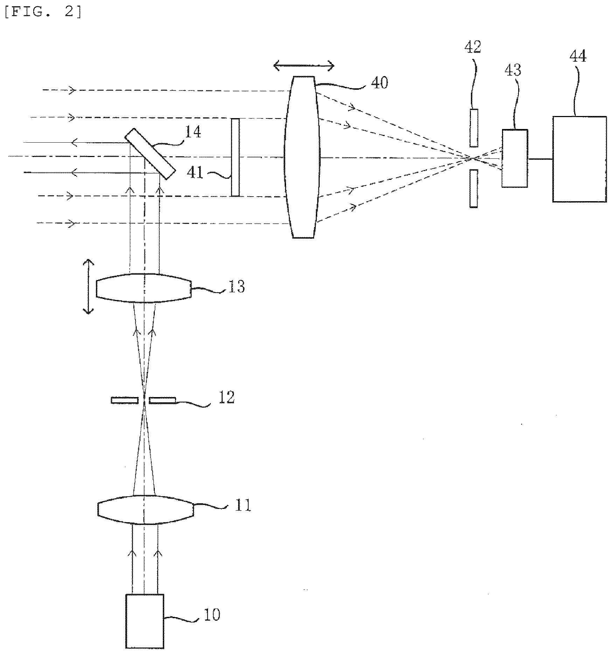

FIG. 2 is an enlarged optical diagram illustrating a light projecting optical system and a light receiving optical system of FIG. 1.

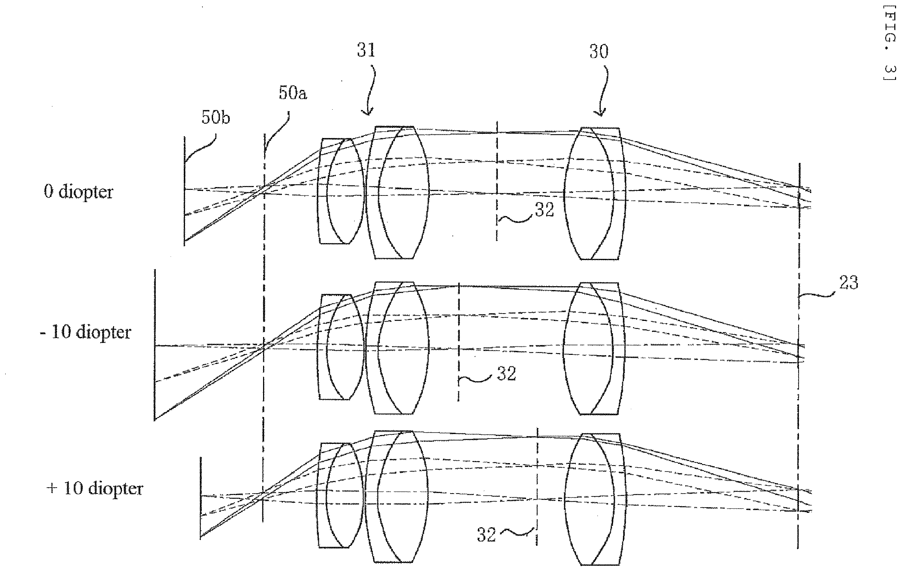

FIG. 3 is an explanatory diagram for explaining that when the diopter adjustment is within a predetermined range, the ocular fundus conjugate plane is located between first and second lens groups of the objective lens optical system.

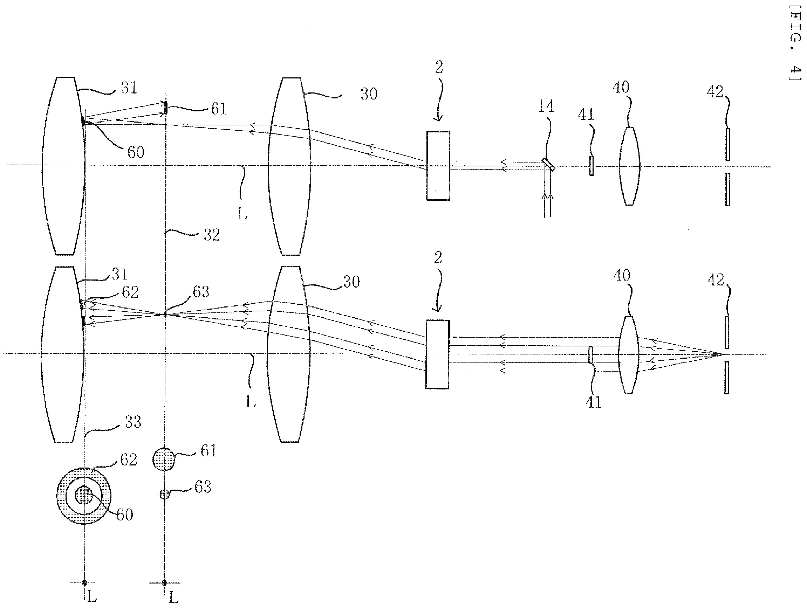

FIG. 4 is an explanatory diagram illustrating a state in which the light beam of scanned illumination light and the light beam of scanned back-projection light are separated on a lens plane of the second lens group of the objective lens optical system while the light beam of reflected light of the scanned illumination light from the same lens plane and the light beam of the scanned back-projection light are separated on the ocular fundus conjugate plane.

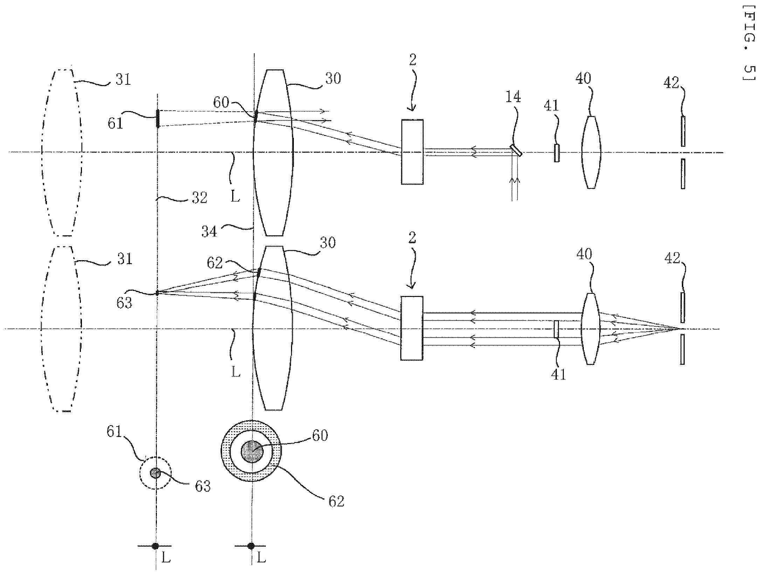

FIG. 5 is an explanatory diagram illustrating a state in which the light beam of scanned illumination light and the light beam of scanned back-projection light are separated on a lens plane of the first lens group of the objective lens optical system while the light beam of reflected light of the scanned illumination light from the same lens plane and the light beam of the scanned back-projection light overlap each other on the ocular fundus conjugate plane.

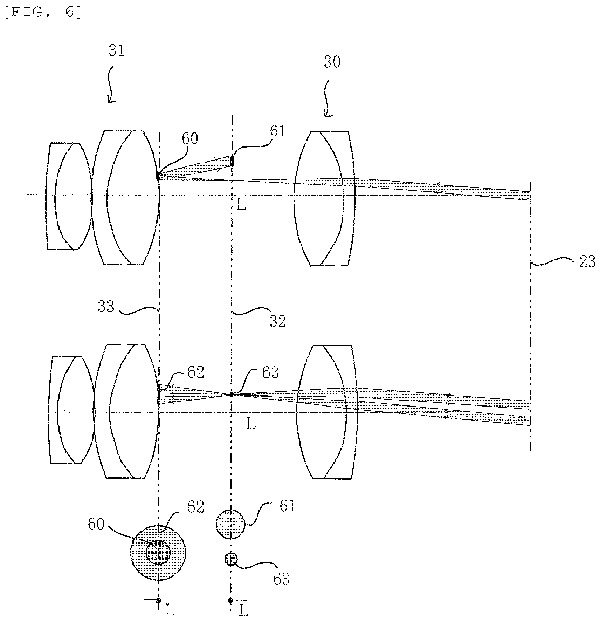

FIG. 6 is an explanatory diagram illustrating a state in which the light beam of scanned illumination light and the light beam of scanned back-projection light overlap each other on a lens plane of the second lens group of the objective lens optical system while the light beam of reflected light of the scanned illumination light from the same lens plane and the light beam of the scanned back-projection light are separated on the ocular fundus conjugate plane.

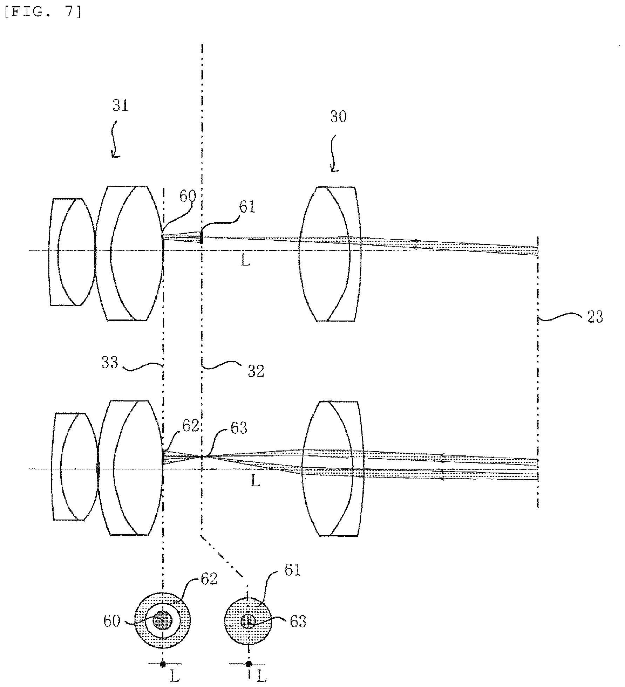

FIG. 7 is an explanatory diagram illustrating a state in which the light beam of scanned illumination light and the light beam of scanned back-projection light are separated on a lens plane of the second lens group of the objective lens optical system while the light beam of reflected light of the scanned illumination light from the same lens plane and the light beam of the scanned back-projection light overlap each other on the ocular fundus conjugate plane.

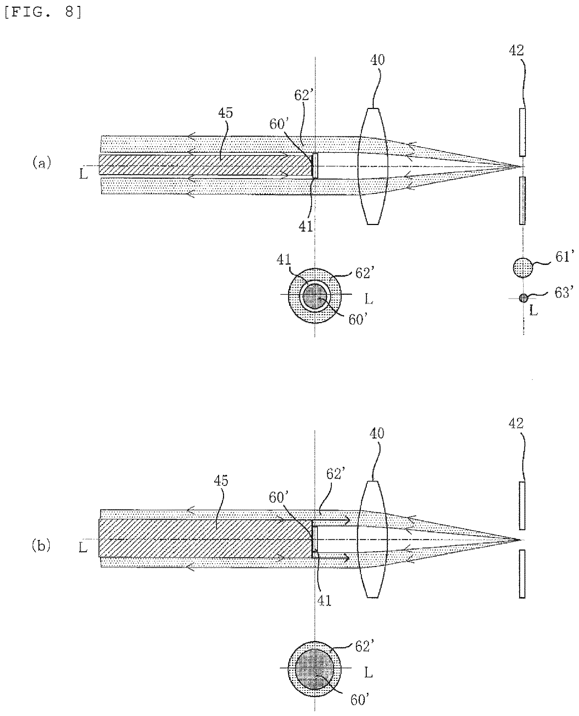

FIG. 8(a) is an explanatory diagram illustrating a state in which harmful reflected light is shielded by the light shielding member and FIG. 8(b) is an explanatory diagram illustrating a state in which harmful reflected light is not shielded by the light shielding member and part of the harmful reflected light enters the light receiving pinhole.

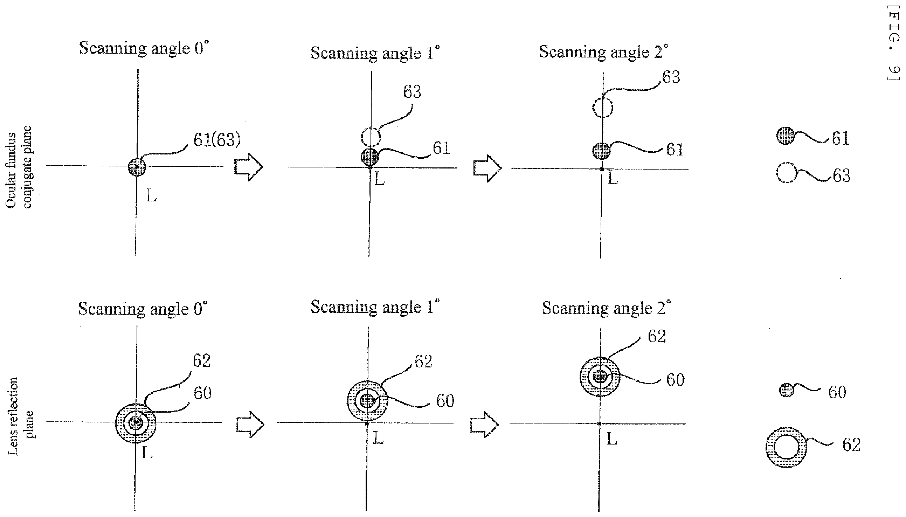

FIG. 9 is an explanatory diagram illustrating respective light beams on the ocular fundus conjugate plane and on a lens reflection plane of the objective lens optical system when the scanning angle is changed in a state in which the ocular fundus conjugate plane is sufficiently separated from lens planes of the objective lens optical system.

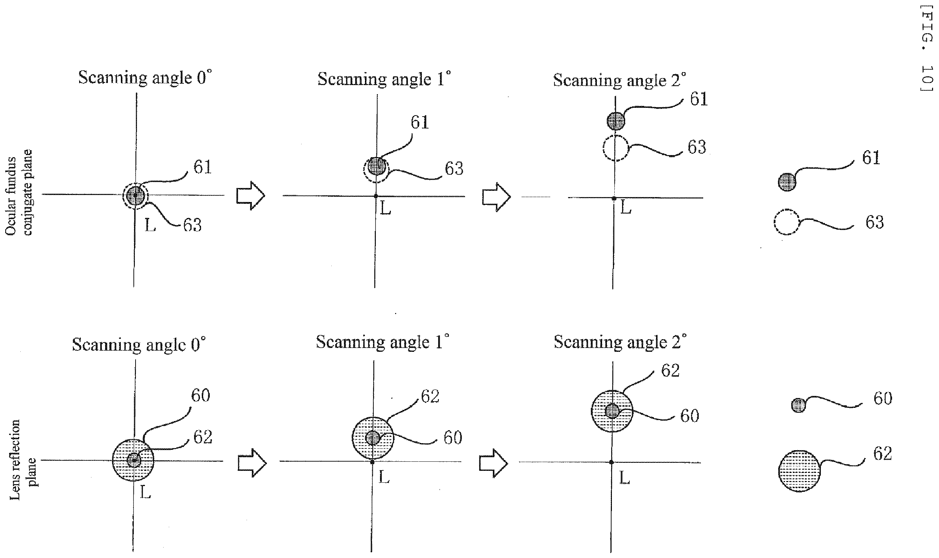

FIG. 10 is an explanatory diagram illustrating respective light beams on the ocular fundus conjugate plane and on a lens reflection plane of the objective lens optical system when the scanning angle is changed in a state in which the ocular fundus conjugate plane is close to lens planes of the objective lens optical system.

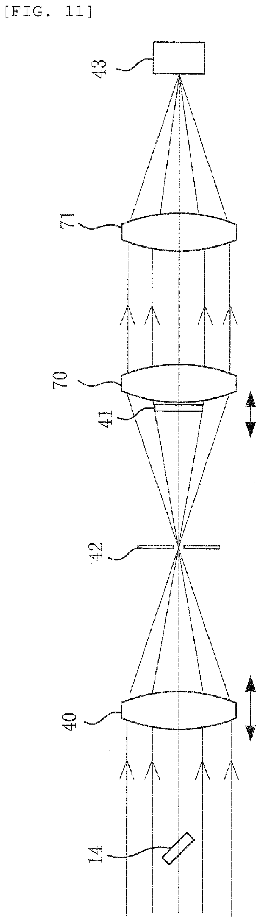

FIG. 11 is an optical diagram illustrating another example of the light receiving optical system.

### [Embodiments for Carrying out the Invention]

Hereinafter, the present invention will be described in detail on the basis of one or more examples or embodiments with reference to the drawings.

### [Example]

FIG. 1 illustrates the overall configuration of a scanning type ocular fundus imaging device according to the present example and FIG. 2 is an enlarged diagram illustrating a light projecting optical system and a light receiving optical system. In each figure, laser light from a laser light source 10 passes through a condenser lens 11 and is incident on a light projecting pinhole 12 disposed at a position conjugate with an ocular fundus conjugate plane. The laser light having a diameter reduced by the light projecting pinhole 12 is converted into a parallel light beam by a collimator lens 13 and is incident on an optical path splitting mirror (pupil division mirror) 14. The condenser lens 11, the light projecting pinhole 12, and the collimator lens 13 constitute a light projecting optical system 1 that projects the light emitted from the laser light source 10 as illumination light to an ocular fundus 50b through a pupil 50a of a subject eye 50.

The laser light incident on the optical path splitting mirror 14 is reflected therefrom and incident on a horizontal scanning device 20 and is scanned at high speed in the horizontal direction (main scanning direction). The horizontal scanning device 20 is composed, for example, of a polygon mirror. The laser light scanned at high speed is incident on a vertical scanning device 23 via scanning relay lenses 21 and 22 and scanned at low speed in the vertical direction (sub-scanning direction). The vertical scanning device 23 is composed, for example, of a galvanomirror. The horizontal scanning device 20, the scanning relay lenses 21 and 22, and the vertical scanning device 23 constitute a scanning optical system 2.

The laser light scanned in the horizontal and vertical directions by the scanning optical system 2 is incident on an objective lens optical system 3 that is composed of a first lens group (which may be a single lens) 30 and a second lens group (which may be a single lens) 31. As illustrated in FIG. 3, the second lens group 31 is disposed on the side close to the subject eye 50 so that the pupil 50a of the subject eye 50 is positioned in the vicinity of the back focal point. On the other hand, the first lens group 30 is disposed on the side distant from the subject eye 50, and the vertical scanning device 23 located at a position conjugate with the pupil 50a of the subject eye 50 is disposed in the vicinity of the front focal point.

The laser light scanned two-dimensionally by the scanning devices 20 and 23 is incident as scanned illumination light on the pupil 50a of the subject eye 50 via the objective lens optical system 3 and projected on the ocular fundus 50b. The ocular fundus 50b is raster-scanned with the laser light in the horizontal and vertical directions, and the ocular fundus 50b in the scanning area is illuminated with the laser light.

The laser light projected on the ocular fundus 50b is reflected from the ocular fundus 50b, and the reflected light travels in the same optical path in the opposite direction and passes through the objective lens optical system 3. The objective lens optical system 3 is configured such that, as illustrated in FIGS. 1 and 3, an ocular fundus conjugate plane 32 exists between the first lens group 30 and the second lens group 31, and an ocular fundus image is formed on the ocular fundus conjugate plane 32.

The laser light having passed through the objective lens optical system 3 is reversely scanned in the vertical and horizontal directions by the scanning optical system 2 and incident on the optical path splitting mirror 14 as the same light ray as before being incident on the scanning optical system 2. The optical path splitting mirror 14, which is disposed so that its center coincides with the optical axis, passes the reflected light from outside of the mirror to the light receiving optical system 4 and therefore splits the optical path into a light projecting optical path and a light receiving optical path. On the other hand, the optical path on the subject eye side of the optical path splitting mirror 14 is an optical path common to the light projecting optical system 1 and the light receiving optical system 4.

The light receiving optical system 4 is composed of a light receiving lens 40, a light shielding member 41, a light receiving pinhole 42, and a light receiving element 43. The reflected light from the ocular fundus 50b having passed through the optical path splitting mirror 14 passes through the light shielding member 41, the light receiving lens 40, and the light receiving pinhole 42 and is received by the light receiving element 43. The light receiving pinhole 42 is disposed in the vicinity of a position conjugate with the ocular fundus 50b. The light shielding member 41 is disposed in the vicinity of a position conjugate with the anterior ocular part, for example, the pupil or the crystalline lens, of the subject eye and shields the harmful reflected light from a lens plane of the objective lens optical system 3.

The light receiving element 43, which is composed of a photodiode, for example, sends luminance information at each point of the raster-scanned ocular fundus 50b to an image processing unit 44. The image processing unit 44 creates an ocular fundus image from the scanning position of the ocular fundus 50b and its luminance information. Although not illustrated, the ocular fundus image thus created is stored in a storage device, displayed on a display, or printed by a printer.

In the present example, a diopter adjusting mechanism for diopter adjustment of the subject eye 50 is provided in each of the light projecting optical system 1 and the light receiving optical system 4. As illustrated in FIGS. 1 and 2, in the light projecting optical system 1, the collimator lens 13 is used as a focus lens for the diopter adjustment, while in the light receiving optical system 4, the light receiving lens 40 is used as a focus lens for the diopter adjustment. The collimator lens 13 and the light receiving lens 40 are moved by the same amount along the optical axis in association with each other thereby to perform the diopter adjustment.

If the ocular fundus conjugate plane 32 overlaps a lens plane of the objective lens optical system 3, the occurrence of a center spot image (false image) that causes harmful light due to reflection from the objective lens plane cannot be avoided. In the objective lens optical system 3 of the present example, therefore, the first and second lens groups 30 and 31 are disposed at a distance from each other in the optical axis direction so that the ocular fundus conjugate plane 32 exists in the space therebetween. Thus, even when the objective lens is composed of a plurality of lens groups, the ocular fundus conjugate plane does not overlap an objective lens plane, and the occurrence of a center spot image can be prevented.

During the diopter adjustment, the ocular fundus conjugate plane 32 moves along the optical axis in the space between the first lens group 30 and the second lens group 31 and may possibly overlap a lens plane of the first or second lens group depending on the diopter adjustment. In the present example, therefore, the distance between the first lens group 30 and the second lens group 31 is set such that the ocular fundus conjugate plane 32 is located at a position that is not close to or does not overlap a lens plane of the first or second lens group. As illustrated in FIG. 3, in the case of a subject eye having a normal diopter of 0 diopter, the ocular fundus conjugate plane 32 is located substantially at the center between the first and second lens groups 30 and 31. Additionally or alternatively, in the case of a subject eye of -10 diopter, the ocular fundus conjugate plane 32 is located on the second lens group 31 side. Additionally or alternatively, in the case of a subject eye of +10 diopter, the ocular fundus conjugate plane 32 is located on the first lens group 30 side.

In such a configuration, when capturing an image of the ocular fundus, the laser light source 10 is turned on. The light from the laser light source 10 is split in the optical path by the optical path splitting mirror 14 via the light projecting optical system 1 and scanned two-dimensionally in the horizontal and vertical directions by the scanning optical system 2, passes through the objective lens optical system 3, and is projected as the scanned illumination light on the ocular fundus 50b of the subject eye 50.

The ocular fundus 50b of the subject eye 50 is raster-scanned with the laser light, and the reflected light from the ocular fundus 50b passes through the objective lens optical system 3, the scanning optical system 2, and the optical path splitting mirror 14 and is incident on the light receiving optical system 4. Most of the harmful reflected light is removed by the optical path splitting mirror 14, and the harmful reflected light from an objective lens plane is shielded by the light shielding member 41 and the light shielding part of the light receiving pinhole 42. This allows the light receiving element 43 to receive the reflected light from the ocular fundus without a center spot image (false image due to harmful light). The light receiving element 43 sends the luminance information of each point of the raster-scanned ocular fundus 50b to the image processing unit 44, which creates an ocular fundus image from the scanning position of the ocular fundus 50b and its luminance information and provides the captured image of the ocular fundus.

When adjusting the diopter of the subject eye, the collimator lens 13 of the light projecting optical system and the light receiving lens 40 of the light receiving optical system 4 are moved in association with each other thereby to adjust the diopter. The diopter adjustment is usually performed within a range of -10 diopter to +10 diopter. In the diopter adjustment within this range, as illustrated in FIG. 3, the ocular fundus conjugate plane 32 is located in the space between the first lens group 30 and the second lens group 31; therefore, the ocular fundus conjugate plane 32 does not overlap a lens plane of the first or second lens group and it is thus possible to prevent the occurrence of a center spot image due to harmful reflected light from the objective lens plane.

Moreover, the diopter adjusting mechanism is provided in each of the light projecting optical system and the light receiving optical system rather than on the common optical path of these systems, and the diopter adjustment can therefore be reliably performed because it is not necessary to incline the diopter adjusting lenses with respect to the optical axis to avoid the occurrence of a center spot image.

In the present example, as illustrated in FIGS. 4 to 7, at least one of the following two conditions should be satisfied to further reliably prevent the occurrence of a center spot image in the captured image of an ocular fundus. The first condition is that the light beam of scanned back-projection light exiting the light receiving pinhole 42 and passing through the light shielding member 41 and the scanning optical system 2 and the light beam of the scanned illumination light passing through the same scanning optical system 2 are separated on the same lens plane of the objective lens optical system 3. The second condition is that the light beam of reflected light of the scanned illumination light from the same lens plane and the light beam of the scanned back-projection light from the light receiving pinhole 42 passing through the same scanning optical system 2 are separated on the ocular fundus conjugate plane 32. FIG. 4 illustrates a state that simultaneously satisfies the above two conditions, which is the best condition for reliably preventing the occurrence of a center spot image.

FIGS. 4, 6, and 7 each illustrate a case in which the scanned illumination light scanned by the scanning optical system 2 is reflected from a lens plane of the second lens group 31 of the objective lens optical system 3 while FIG. 5 illustrates a case in which the scanned illumination light is reflected from a lens plane of the first lens group 30.

The upper part of FIG. 4 illustrates a state in which a simulation is conducted such that laser light is generated and the generated laser light is reflected from the optical path splitting mirror 14 and two-dimensionally scanned by the scanning optical system 2 to become the scanned illumination light for illuminating the ocular fundus. The scanned illumination light passes through the first lens group 30 of the objective lens optical system 3 and reaches the second lens group 31, and the reflected light from a lens plane of the second lens group 31 passes through the ocular fundus conjugate plane 32. As illustrated in the lower part of the figure, when viewed from a direction orthogonal to the optical axis L, both the light beam 60 of the scanned illumination light on a lens plane 33 of the second lens group 31 and the light beam 61 on the ocular fundus conjugate plane 32 of reflected light of the scanned illumination light from the same lens plane 33 are located above the optical axis L.

On the other hand, the middle part of FIG. 4 illustrates a state in which a simulation is conducted such that scanned back-projection light is generated by being scanned using the same scanning optical system 2 as for the scanned illumination light after passing through the light receiving lens 40 and the light shielding member 41 from the light receiving pinhole 42 as an object point. The scanned back-projection light passes through the ocular fundus conjugate plane 32 of the objective lens optical system 3 and reaches the lens plane 33 of the second lens group 31. As illustrated in the lower part of FIG. 4, the scanned back-projection light obtained by the simulation becomes a light beam 63 on the ocular fundus conjugate plane 32 and a light beam 62 on the lens plane 33 of the second lens group 31, both of which pass through above the optical path L.

The light beams 61 and 63 on the ocular fundus conjugate plane 32 and the light beams 60 and 62 on the lens plane of the objective lens optical system illustrated in the lower part of FIG. 4 and the lower parts of FIGS. 5 to 7 are each an image rendered by the simulation and are illustrated in an enlarged manner. The lens planes of the objective lens optical system are denoted by reference numeral 33 in FIGS. 4, 6, and 7 and by reference numeral 34 in FIG. 5. These lens planes are illustrated as flat surfaces orthogonal to the optical axis L for descriptive purposes, but actually curved in accordance with the curvature of each lens plane, and the light beams 60 and 62 on the lens planes 33 and 34 illustrated in the lower parts of respective figures represent the images on the curved planes.

Referring again to the lower part of FIG. 4, in the light beam 62 of the scanned back-projection light on the lens plane 33 of the objective lens optical system 3, a circular shadow corresponding to the shape of the light shielding member 41 appears inside the light beam 62. The light beam 62 therefore has a ring shape in which the central part is a circular dark part (shadow) and its circumference is a bright part. In each figure, the bright parts of the light beams 60 to 63 are illustrated with shading and the dark parts are illustrated in white, so the bright and dark parts of each light beam are illustrated in reverse to the actual ones.

In the example illustrated in FIG. 4, as illustrated in the lower part, the light beam of the scanned back-projection light and the light beam of the scanned illumination light, which have passed through the scanning optical system 2, are respectively the light beams 62 and 60 on the lens plane 33 of the objective lens optical system 3. Here, the light beam 60 is in the shadow part (white part) of the light beam 62 and the two light beams 60 and 62 are separated; therefore, the first condition is satisfied. Likewise, the light beam of the scanned back-projection light and the light beam of reflected light of the scanned illumination light from the same lens plane 33, which have passed through the scanning optical system 2, are respectively the light beams 63 and 61 on the ocular fundus conjugate plane 32, and the two light beams are separated; therefore, the second condition is satisfied.

As illustrated in FIG. 8(a), the images of the light beams 60 and 62 on the lens plane 33 correspond respectively to a light beam 60' and a light beam 62' on the light shielding member 41. The light beam 60' corresponds to the light beam of harmful reflected light 45 from the lens plane 33, which is incident along the optical path L and indicated by oblique lines, while the light beam 62' corresponds to the light beam of the back-projection light on the light shielding member 41. When the light beams 60 and 62 on the lens plane 33 are separated as illustrated in FIG. 4, the light beams 60' and 62' at the position of the light shielding member 41 are also separated as illustrated in FIG. 8(a). This indicates that the harmful reflected light 45 is entirely shielded by the light shielding member 41 and is not incident on the light receiving pinhole 42, and the occurrence of a center spot image is prevented. In contrast, when the light beams 60 and 62 overlap each other on the lens plane 33, the light beams 60' and 62' also overlap each other as illustrated in FIG. 8(b). This indicates that the harmful reflected light 45 cannot be entirely shielded by the light shielding member 41 and part of the harmful reflected light 45 passes through the light shielding member 41 and is incident on the light receiving pinhole 42, as indicated by arrows, and a center spot image occurs. Thus, the light beams 60 and 62 being separated on the lens plane 33 is the first condition that a center spot image does not occur.

On the other hand, when the light beams 63 and 61 are separated on the ocular fundus conjugate plane 32, light beams 63' and 61' on the light receiving pinholes 42 corresponding to the light beams 63 and 61 are also separated because the light receiving pinhole 42 is on the ocular fundus conjugate plane. The light beam 63' forms an ocular fundus image while the light beam 61' is the harmful reflected light from the lens reflection plane and therefore shielded by the light shielding part of the light receiving pinhole 42. Accordingly, the light beams 63 and 61 being separated on the ocular fundus conjugate plane 32 is the second condition that a center spot image does not occur. Thus, two conditions of the first and second conditions being satisfied in the simulation means that the harmful reflected light generated due to the reflection of the scanned illumination light from the same lens plane can be shielded by the combination of the light shielding member 41 and the light receiving pinhole 42.

Even when both the above-described first and second conditions are not satisfied, that is, when only one of the conditions is satisfied, it is possible to shield the harmful reflected light generated due to the reflection from a lens plane of the objective lens optical system. Accordingly, parameters such as the hole diameter of the light receiving pinhole 42 of the light receiving optical system, the diameter of the light shielding plane of the light shielding member 41, and the power distribution and lens shapes of the objective lens optical system are set such that a state is made in which the light beam of the scanned back-projection light and the light beam of the scanned illumination light do not overlap each other on a lens plane of the objective lens optical system or a state is made in which the light beam of the scanned back-projection light and the light beam of the reflected light of the scanned illumination light from the same lens plane do not overlap each other on the ocular fundus conjugate plane.

Additionally or alternatively, the above-described first condition or second condition should be satisfied in the entire range of the diopter adjustment by the diopter adjusting mechanism. For example, if the ocular fundus conjugate plane 32 is located at a position as illustrated in FIG. 6 due to the diopter adjustment, the light beams 62 and 60 overlap each other on the lens plane 33, so that the first condition is not satisfied. However, even if the first condition is not satisfied, as illustrated on the right side of the lower part of FIG. 6, when the lens shapes of the second lens group 31, the shape of the light shielding member 41, and/or the shape of the light receiving pinhole 42 (e.g., the hole diameter of the light receiving pinhole 42) are set such that the light beam 61 of the reflected light of the scanned illumination light from the lens plane 33 is separated from the light beam 63 of the scanned back-projection light on the ocular fundus conjugate plane 32 to satisfy the second condition, a center spot image (false image due to harmful reflected light) does not occur.

On the other hand, FIG. 7 illustrates a state in which the ocular fundus conjugate plane 32 is located at a position closer to the second lens group 31 than that in the state illustrated in FIG. 6. In the example illustrated in FIG. 7, the light beam 60 of the scanned illumination light and the light beam 62 of the scanned back-projection light are separated on the lens plane 33 of the second lens group 31, but the light beam 61 of the reflected light of the scanned illumination light from the lens plane 33 and the light beam 63 of the scanned back-projection light are not separated on the ocular fundus conjugate plane 32. In this case, a center spot image does not occur because the first condition is satisfied, but the light beams 61 and 63 can also be separated by changing the lens shapes of the second lens group 31, the shape of the light shielding member 41, and the like. Thus, the optimum solution is obtained by satisfying the condition that the light beams 60 and 62 are separated or the light beams 61 and 63 are separated and minimizing the diameter of the light shielding member 41.

The above-described first condition that the two light beams 60 and 62 are separated on the lens plane 33 of the objective lens optical system 3 or the second condition that the two light beams 61 and 63 are separated on the ocular fundus conjugate plane 32 should be satisfied at each scanning angle (scanning point) of the scanning optical system 2. The upper part of FIG. 9 illustrates the separation state of the light beam 61 and the light beam 63 on the ocular fundus conjugate plane 32, and the lower part of FIG. 9 illustrates the separation state of the light beam 60 and the light beam 62 on the lens reflection plane (lens plane 33 of the objective lens optical system 3). Both the separation states are obtained after the light beams pass through the same scanning optical system 2, and the positional relationship between the light beam images is illustrated under different conditions of scanning angles of 0°, 1°, and 2°. At the scanning angle of 0°, the light beam 61 and the light beam 63 overlap each other on the ocular fundus conjugate plane, but the light beam 60 and the light beam 62 are separated on the lens reflection plane, so a center spot image does not occur. At the scanning angles of 1° and 2°, the light beam 61 and the light beam 63 are separated on the ocular fundus conjugate plane, and the light beam 60 and the light beam 62 are also separated on the lens reflection plane, so a center spot image does not occur.

While FIG. 9 illustrates the setting in which the ocular fundus conjugate plane is sufficiently separated from a lens plane of the objective lens optical system, FIG. 10 illustrates an example of the case in which the ocular fundus conjugate plane is close to the lens plane of the objective lens optical system. At both the scanning angle of 0° and the scanning angle of 1°, the light beam 61 and the light beam 63 overlap each other on the ocular fundus conjugate plane, and the light beam 60 and the light beam 62 also overlap each other on the lens reflection plane. On the other hand, at the scanning angle of 2°, the light beam 60 and the light beam 62 overlap each other on the lens reflection plane, but the light beam 61 and the light beam 63 are separated on the ocular fundus conjugate plane. Thus, a center spot image occurs under the conditions of the scanning angles of 0° and 1°, and in this case, it is found that the design of the objective lenses may have to be reconsidered so that the ocular fundus conjugate plane is relocated away from the lens plane of the objective lens optical system 3.

Regarding the size of the light shielding member 41, it is preferred to set the size ratio between the light beam image of the scanned illumination light and the image of the light shielding member 41 on the pupil plane of the pupil 50a of the subject eye within a range of 1:1 to 1:4. As the ratio becomes smaller, the optical system can capture an image even with a small pupil diameter, which is advantageous in capturing the image of an ocular fundus, but on the other hand, a center spot image is more likely to occur. In contrast, as the ratio becomes larger, a center spot image is less likely to occur, which is advantageous in removing a false image, but there is a disadvantage that a bright fundus image cannot be obtained for a patient with a small pupil diameter.

The harmful reflected light (center spot image) is generated not only on the lens plane 33 of the second lens group 31 as described above but also on the lens plane 34 of the first lens group 30 as illustrated in FIG. 5. In this case, the light beam of the reflected light of the scanned illumination light from the lens plane 34 of the first lens group 30 is a light beam 61 of a virtual image on the ocular fundus conjugate plane 32 while the light beam of the scanned back-projection light is a light beam 63 of a real image on the ocular fundus conjugate plane 32. As illustrated in the lower part of the figure, the two light beams 61 and 63 overlap each other on the ocular fundus conjugate plane 32 and the second condition is not satisfied. As illustrated in the lower part of the figure, therefore, the light beam 60 of the scanned illumination light and the light beam 62 of the scanned back-projection light are separated on the lens plane 34 of the first lens group 30 to satisfy the first condition by disposing the first lens group 30 at a distance sufficiently separated from the ocular fundus conjugate plane 32 and/or setting the shape and the like of the light shielding member 41.

Thus, even if the two light beams 61 and 63 cannot be separated on the ocular fundus conjugate plane 32, when the two light beams 60 and 62 do not overlap each other on the lens plane 34 of the objective lens optical system, the occurrence of a center spot image can be reliably prevented.

When the diopter is adjusted in the minus direction, the ocular fundus conjugate plane comes close to the second lens group 31 and a center spot image is likely to occur. To prevent this, in the case of the diopter adjustment in the minus direction, the light shielding member may have to be made larger than that at the case of the 0 diopter. However, it is difficult to continuously change the size of the light shielding member in accordance with the diopter. An example for solving this problem is illustrated in FIG. 11.

In FIG. 11, the light shielding member 41 is disposed closer to the light receiving element 43 than the light receiving pinhole 42, and the reflected light from the ocular fundus having passed through the light receiving pinhole 42 passes through the light shielding member 41 and is relayed by light receiving relay lenses 70 and 71 to form an image on the light receiving element 43. The light receiving lens 40 is moved to come close to the light receiving pinhole 42 in the minus diopter direction and moved away from the light receiving pinhole 42 in the plus diopter direction.

Additionally or alternatively, in FIG. 11, the light shielding member 41 is configured as follows. During the diopter adjustment by the light receiving lens 40, even when the light receiving lens 40 is moved within an adjustment range from the plus diopter to the 0 diopter, the light shielding member 41 is not moved, while during the adjustment to the minus diopter, the light shielding member 41 is moved toward the light receiving pinhole 42 side in a direction opposite to the moving direction of the light receiving lens 40.

In such a configuration, in a normal diopter adjustment range, as described above, the ocular fundus conjugate plane 32 formed by the objective lens optical system is located between the first and second lens groups 30 and 31. Most of the harmful reflected light is removed by the optical path splitting mirror 14 and the light receiving pinhole 42, and the harmful reflected light from an objective lens plane having passed through the light receiving pinhole 42 is shielded by the light shielding member 41. At this time, when the adjustment to the minus diopter is performed, the light shielding member 41 is continuously moved toward the light receiving pinhole 42 side in the direction opposite to the moving direction of the light receiving lens 40, and the apparent light shielding size increases accordingly to respond to a large center spot image. This allows the same effect to be obtained as that when the light shielding member 41 is fixed and its size is continuously changed, without changing the size of the light shielding member 41.

### [Description of Reference Numerals]

- 1: Light projecting optical system
- 2: Scanning optical system
- 3: Objective lens optical system
- 4: Light receiving optical system
- 10: Laser light source
- 11: Condenser lens
- 12: Light projecting pinhole
- 13: Collimator lens
- 14: Optical path splitting mirror
- 20: Horizontal scanning device
- 21, 22: Scanning relay lens
- 23: Vertical scanning device
- 30: First lens group
- 31: Second lens group
- 32: Ocular fundus conjugate plane
- 40: Light receiving lens
- 41: Light shielding member
- 42: Light receiving pinhole
- 43: Light receiving element
- 44: Image processing unit
- 50: Subject eye
- 50a: Pupil
- 50b: Ocular fundus
- 70, 71: Light receiving relay lens

## Claims

1. A scanning type ocular fundus imaging device comprising:
a light projecting optical system that projects light from a light source on an ocular fundus of a subject eye via an objective lens optical system, the light being scanned as scanned illumination light using a scanning optical system;
a light receiving optical system that receives reflected light from the ocular fundus via the objective lens optical system and the scanning optical system;
a diopter adjusting mechanism that moves an ocular fundus conjugate plane formed by the objective lens optical system in an optical axis direction;
a light receiving pinhole disposed in a vicinity of an ocular fundus conjugate position in the light receiving optical system; and
a light shielding member disposed in the light receiving optical system to shield harmful reflected light from a lens plane of the objective lens optical system,
wherein the objective lens optical system is composed of a first lens group and a second lens group disposed at a distance from the first lens group in the optical axis direction, and the ocular fundus conjugate plane is located between the first and second lens groups of the objective lens optical system within a diopter adjustment range by the diopter adjusting mechanism.

2. The scanning type ocular fundus imaging device according to claim 1, wherein the light shielding member is disposed in a vicinity of a position conjugate with an anterior ocular part of the subject eye.

3. The scanning type ocular fundus imaging device according to claim 1 or 2, wherein the diopter adjusting mechanism is one of respective diopter adjusting mechanisms provided for the light projecting optical system and the light receiving optical system, and the diopter adjusting mechanisms operate in association with each other.

4. The scanning type ocular fundus imaging device according to any one of claims 1 to 3, wherein the diopter adjustment range is a range from -10 diopter to +10 diopter.

5. The scanning type ocular fundus imaging device according to claim 1, wherein the light shielding member is disposed closer to a light receiving element than the light receiving pinhole.

6. The scanning type ocular fundus imaging device according to claim 5, wherein the light shielding member is moved toward the light receiving pinhole side in accordance with diopter adjustment.

7. The scanning type ocular fundus imaging device according to claim 6, wherein the diopter adjustment is adjustment from 0 diopter to minus diopter side.

8. The scanning type ocular fundus imaging device according to any one of claims 1 to 7, wherein the light receiving optical system and the objective lens optical system are configured such that a light beam of scanned back-projection light is separated on the lens plane of the objective lens optical system from a light beam of the scanned illumination light, wherein the scanned back-projection light is scanned by the scanning optical system after exiting the light receiving pinhole and passing through the light shielding member, wherein same scanning as that for the scanned back-projection light is performed for the scanned illumination light.

9. The scanning type ocular fundus imaging device according to any one of claims 1 to 7, wherein the light receiving optical system and the objective lens optical system are configured such that a light beam of scanned back-projection light is separated on the ocular fundus conjugate plane from a light beam of reflected light of the scanned illumination light from the lens plane of the objective lens optical system, wherein the scanned back-projection light is scanned by the scanning optical system after exiting the light receiving pinhole and passing through the light shielding member, wherein same scanning as that for the scanned back-projection light is performed for the scanned illumination light.

10. A scanning type ocular fundus imaging device comprising:
a light projecting optical system that projects light from a light source on an ocular fundus of a subject eye via an objective lens optical system, the light being scanned as scanned illumination light using a scanning optical system;
a light receiving optical system that receives reflected light from the ocular fundus via the objective lens optical system and the scanning optical system;
a light receiving pinhole disposed in a vicinity of an ocular fundus conjugate position in the light receiving optical system; and
a light shielding member disposed in the light receiving optical system to shield harmful reflected light from a lens plane of the objective lens optical system,
wherein the light receiving optical system and the objective lens optical system are configured such that a light beam of scanned back-projection light is separated on the lens plane of the objective lens optical system from a light beam of the scanned illumination light, wherein the scanned back-projection light is scanned by the scanning optical system after exiting the light receiving pinhole and passing through the light shielding member, wherein same scanning as that for the scanned back-projection light is performed for the scanned illumination light.

11. A scanning type ocular fundus imaging device comprising:
a light projecting optical system that projects light from a light source on an ocular fundus of a subject eye via an objective lens optical system, the light being scanned as scanned illumination light using a scanning optical system;
a light receiving optical system that receives reflected light from the ocular fundus via the objective lens optical system and the scanning optical system;
a light receiving pinhole disposed in a vicinity of an ocular fundus conjugate position in the light receiving optical system; and
a light shielding member disposed in the light receiving optical system to shield harmful reflected light from a lens plane of the objective lens optical system,
wherein the light receiving optical system and the objective lens optical system are configured such that a light beam of scanned back-projection light is separated on the ocular fundus conjugate plane from a light beam of reflected light of the scanned illumination light from the lens plane of the objective lens optical system, wherein the scanned back-projection light is scanned by the scanning optical system after exiting the light receiving pinhole and passing through the light shielding member, wherein same scanning as that for the scanned back-projection light is performed for the scanned illumination light.
